# EUROPEAN PATENT SPECIFICATION

(11) **EP 3 527 718 B1**
(45) Date of publication and mention of the grant of the patent: **04.10.2023**
(21) Application number: 16856451.6
(22) Date of filing: 13.10.2016
(51) Int. Cl.: D07B 1/00, A61M 25/00, F16C 1/02

(54) **MULTI-LAYER BODY, MULTI-LAYER HOLLOW BODY, AND CATHETER PROVIDED WITH MULTI-LAYER HOLLOW BODY**
MEHRSCHICHTIGER KÖRPER, MEHRSCHICHTIGER HOHLKÖRPER UND KATHETER MIT MEHRSCHICHTIGEM HOHLKÖRPER
CORPS À COUCHES MULTIPLES, CORPS CREUX À COUCHES MULTIPLES ET CATHÉTER POURVU D'UN CORPS CREUX À COUCHES MULTIPLES

(43) Date of publication of application: 21.08.2019
(73) Proprietor: ASAHI INTECC CO., LTD., Seto-shi, Aichi 489-0071 (JP)
(72) Inventor: HANAOKA Atsuhiro, Aichi 489-0071 (JP)
(74) Representative: Winter, Brandl - Partnerschaft mbB
(86) International application number: PCT/JP2016/080345
(87) International publication number: WO 2018/070008

(56) References cited:
- WO-A1-2014/136937
- JP-A- H0 723 956
- JP-A- H0 949 517
- JP-A- 2002 195 240
- JP-A- 2011 006 803
- JP-A- 2015 051 085
- US-A- 5 052 404
- US-A1- 2016 101 261

## Description

### Field

The present invention relates to a catheter with a multilayer hollow body with a plurality of hollow bodies arranged concentrically.

### Background

Conventionally, there have been variously proposed multilayer bodies including a plurality of concentric layers.

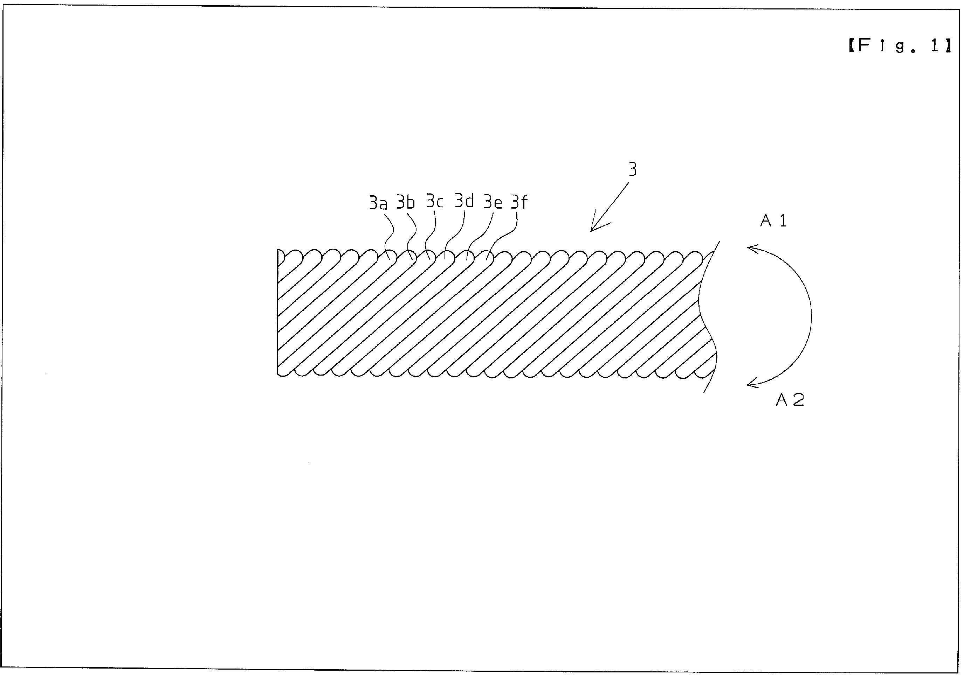

For example, JP 2000 160488 A describes a plural layered and twisted spiral rope (hereinafter, referred to as a "multilayer body") in which S-twisted layers and Z-twisted layers, which are formed by S-twisting or Z-twisting (twist in an opposite direction of S-twist) a plurality of wires around a core wire 6, are arranged alternately in a plurality of layers (see Paragraph [0009] and FIG. 1(c), etc.).

However, in the multilayer body described in JP 2000 160488 A, the stranded wires formed of a plurality of wires are arranged in a plurality of layers around the core wire. Therefore, a stranding angle of the stranded wire in each layer is small, and when one end of the multilayer body is rotated rightward and leftward, the rotational torque transmissivity to the other end has been insufficient.

Moreover, such a problem has occurred similarly not only in a multilayer body but also in a multilayer hollow body formed by removing a core from a multilayer body, and further in a catheter using a multilayer hollow body.

JP 2015 051085 A discloses a cathether in accordance with the preamble of claim 1.

WO 2014 136937 A1 discloses a catheter comprising a tubular body. The tubular body comprises a reinforcing layer, a retention wire and a second reinforcing layer.

US 2016 101261 A1 discloses a catheter comprising multilayer hollow body. The multilayer hollow body comprises a hollow body, an inner braid hollow body and outer hollow body.

Further, US 5052404 A discloses a torque transmitter (guidewire) including three helically wound springs and a medical guide wire assembly including the torque transmitter.

### Summary

### Technical Problem

The present invention has been made to solve the above-described problem, and aims at providing a catheter with a multilayer hollow body having improved rotational torque transmissivity to the other end when one end is rotated rightward and leftward.

### Solution to Problem

The above described technical problem is solved by the subject-matter of Claim 1. Preferred embodiments of the present invention are the subject-matter of dependent claims.

### Advantageous Effects of Invention

The catheter according to the invention includes a multilayer hollow body, a distal end tip that is connected to a distal end of the multilayer hollow body, and a connector that is connected to a proximal end of the multilayer hollow body. The multilayer hollow body includes a single-stripe hollow body wound by one element wire, an inner multi-stripe hollow body that is disposed adjacently to an inner periphery of the single-stripe hollow body and wound by a plurality of element wires in an opposite direction from the single-stripe hollow body, and an outer multi-stripe hollow body that is disposed adjacently to an outer periphery of the single-stripe hollow body and wound by a plurality of element wires in an opposite direction from the single-stripe hollow body. Thus, when one end of the catheter is rotated rightward and leftward, it is possible to improve rotational torque transmissivity to the other end of the catheter.

The catheter according to the present invention may further include an inner layer, wherein the multilayer hollow body is disposed on an outer periphery of the inner layer, an outer layer that covers the inner layer and the multilayer hollow body. The distal end tip may include the distal end of the multilayer hollow body inside the distal end tip and may be connected to a distal end of the inner layer and a distal end of the outer layer. The connector may be connected to a proximal end of the inner layer and a proximal end of the outer layer. Thus, when one end of the catheter is rotated rightward and leftward, it is possible to further improve rotational torque transmissivity to the other end of the catheter.

### Brief Description of Drawings

FIG. 1 is a side view of an inner multi-stripe hollow body of a multilayer hollow body of a catheter according to the invention.
FIG. 2 is a side view of a single-stripe hollow body.
FIG. 3 is a side view of an outer multi-stripe hollow body.
FIG. 4 is an explanatory diagram of a multilayer hollow body.
FIG. 5 is a section explanatory diagram of the multilayer hollow body.
FIG. 6 is a schematic side view of a catheter according to a first embodiment.
FIG. 7 is a schematic side view of a catheter according to a second embodiment.
FIG. 8 is a distal end section view of the catheter according to the second embodiment.

### Description of Embodiments

The following will describe the above-described embodiments of the invention with reference to the enclosed drawings.

### (A multilayer hollow body)

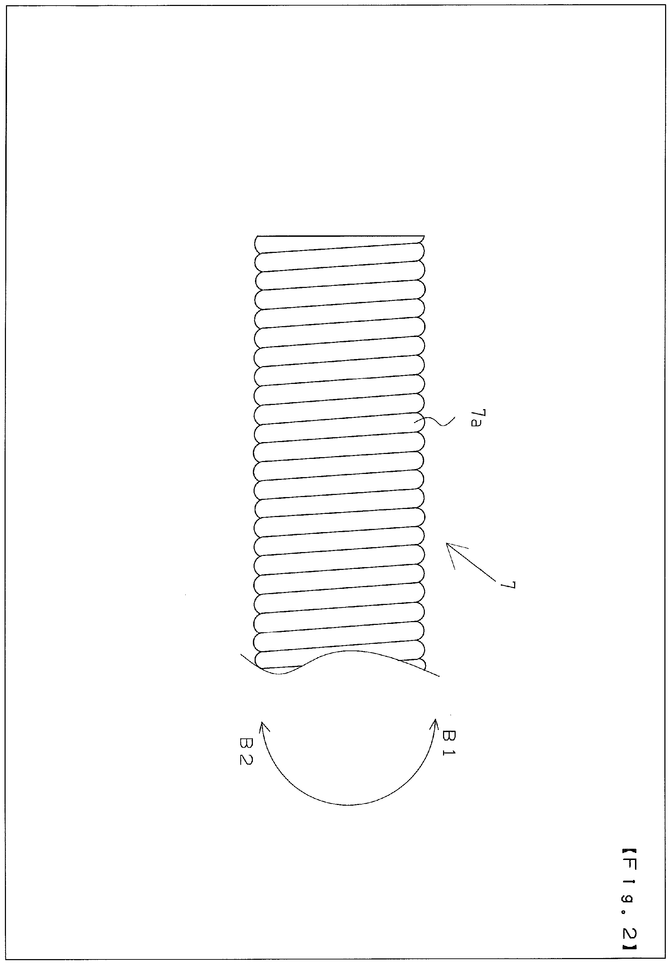

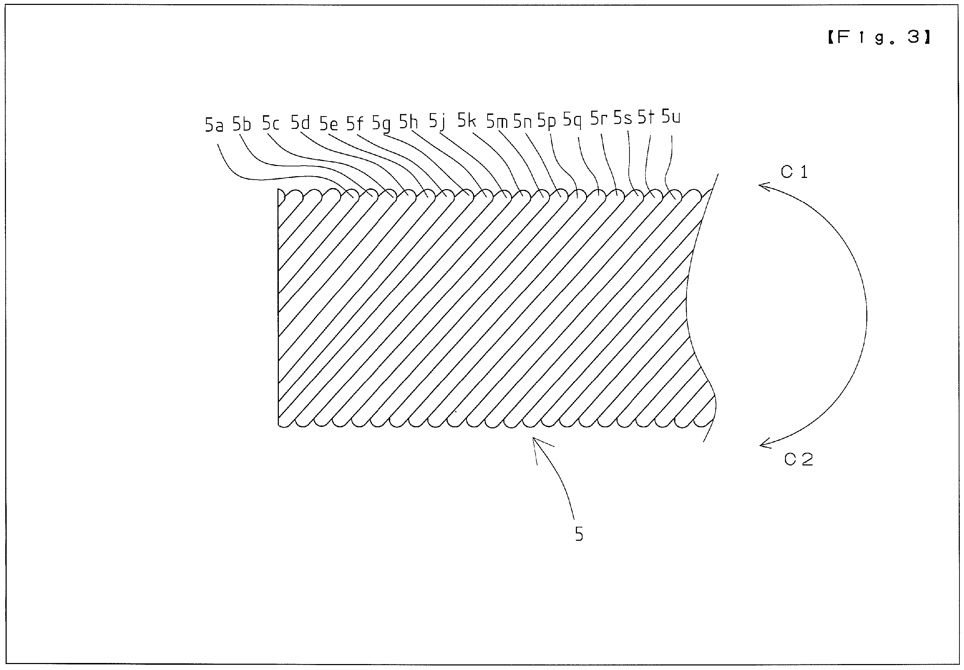

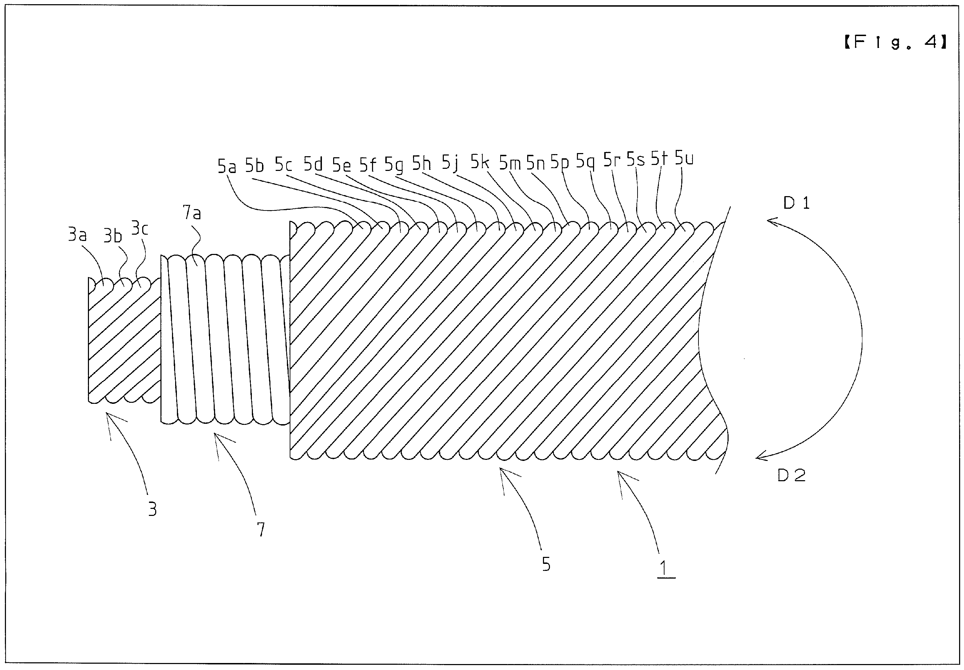

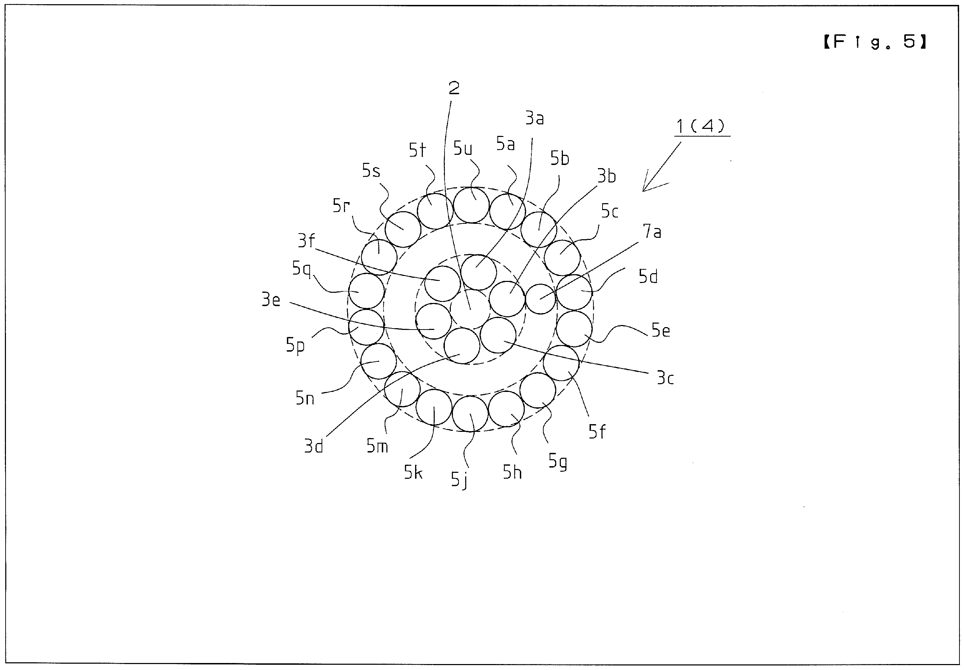

FIG. 1 is a side view of an inner multi-stripe hollow body. FIG. 2 is a side view of a single-stripe hollow body . FIG. 3 is a side view of an outer multi-stripe hollow body. FIG. 4 is an explanatory diagram of the multilayer hollow body. FIG. 5 is a section explanatory diagram of the multilayer hollow body.

In FIG. 1, the inner multi-stripe hollow body 3 is a coil body formed in a hollow form by twisting six element wires.

To be more specific, the inner multi-stripe hollow body 3 is formed in a hollow form by winding metal element wires 3a, 3b, 3c, 3d, 3e, 3f of six stainless alloys.

Note that the inner multi-stripe hollow body 3 is formed by twisting six element wires. However, the embodiment is not limited to one formed by twisting six element wires as long as it is formed by twisting two or more element wires.

Moreover, the material of the element wire forming the inner multi-stripe hollow body 3 is not limited to a stainless alloy, and may be other kinds of metal, e.g., tungsten, platinum, and gold, or resin.

Note that the metal element wires 3a, 3b, 3c, 3d, 3e, 3f forming the inner multi-stripe hollow body 3 are wound in a counterclockwise direction (A2 direction in FIG. 1) toward the left side in the drawing (hereinafter, referred to as a "distal end side").

In FIG. 2, a single-stripe hollow body 7 is a coil body formed in a hollow form by twisting one element wire.

To be more specific, the single-stripe hollow body 7 is formed by winding a metal element wire 7a of one stainless alloy.

Note that the material of the element wire forming the single-stripe hollow body 7 is not limited to a stainless alloy, and may be other kinds of metal, e.g., tungsten, platinum, and gold, or resin.

Note that the metal element wire 7a forming the single-stripe hollow body 7 is wound in a clockwise direction (B1 direction in FIG. 2) toward the left side in the drawing (hereinafter, referred to as a "distal end side").

In FIG. 3, an outer multi-stripe hollow body 5 is a coil body formed in a hollow form by twisting 18 element wires, similarly to the inner multi-stripe hollow body 3.

To be more specific, the outer multi-stripe hollow body 5 is formed by winding metal element wires 5a, 5b, 5c, 5d, 5e, 5f, 5g, 5h, 5j, 5k, 5m, 5n, 5p, 5q, 5r, 5s, 5t, 5u of 18 stainless alloys.

Note that the outer multi-stripe hollow body 5 is formed by twisting 18 element wires. However, the outer multi-stripe hollow body 5 is not limited to one formed by twisting 18 element wires, and it may be formed by twisting two or more element wires as long as it covers the single-stripe hollow body 7. The material of the element wire forming the outer multi-stripe hollow body 5 is not limited to a stainless alloy, and may be other kinds of metals, e.g., tungsten, platinum, gold, or resin.

Note that the metal element wires 5a, 5b, 5c, 5d, 5e, 5f, 5g, 5h, 5j, 5k, 5m, 5n, 5p, 5q, 5r, 5s, 5t, 5u forming the outer multi-stripe hollow body 5 are wound in a counterclockwise direction (C2 direction in FIG. 3) toward the left side in the drawing (hereinafter, referred to as a "distal end side").

FIG. 4 is a diagram explaining the multilayer hollow body 1. In FIG. 4, the inner multi-stripe hollow body 3 and the outer multi-stripe hollow body 5 have winding in the same direction (D2 direction in FIG. 4), and the single-stripe hollow body 7 has winding in an opposite direction to the inner multi-stripe hollow body 3 and the outer multi-stripe hollow body 5 (D1 direction in FIG. 4).

Moreover, in FIG. 4, a twisting angle of the outer multi-stripe hollow body 5 is larger than a twisting angle of the inner multi-stripe hollow body 3, and a twisting angle of the single-stripe hollow body 7 is larger than a twisting angle of the outer multi-stripe hollow body 5.

FIG. 5 is a section explanatory diagram of the multilayer hollow body 1, and illustrates that the outer multi-stripe hollow body 5 is of 18 stripes formed of 18 metal element wires of metal wires 5a, 5b, 5c, 5d, 5e, 5f, 5g, 5h, 5j, 5k, 5m, 5n, 5p, 5q, 5r, 5s, 5t, 5u, that the inner multi-stripe hollow body 3 is of six stripes formed of six metal element wires of metal wires 3a, 3b, 3c, 3d, 3e, 3f, and that the single-stripe hollow body 7 is of one stripe formed of one metal element wire of only metal element wire 7a.

Next, the reason why the rotational torque transmissivity of the multilayer hollow body 1 is improved will be described with reference to FIG. 1 to FIG. 5.

As described above, in the inner multi-stripe hollow body 3 and the outer multi-stripe hollow body 5, the metal element wires are wound in a counterclockwise direction (A2 direction in FIG. 1, C2 direction in FIG. 3) toward the distal end side. In the single-stripe hollow body 7, the metal element wire is wound in a clockwise direction (B1 direction in FIG. 2) toward the distal end side.

### 1. Case of rotating a proximal end of the hollow multilayer body 1 in a clockwise direction (D1 direction in FIG. 4)

In the inner multi-stripe hollow body 3, the metal element wires are wound in a counterclockwise direction (A2 direction in FIG. 1) toward the distal end side. Thus, when the proximal end of the inner multi-stripe hollow body 3 is rotated in a clockwise direction (A1 direction in FIG. 1), an outer diameter of the inner multi-stripe hollow body 3 becomes small.

Also in the outer multi-stripe hollow body 5, the metal element wires are wound in a counterclockwise direction (C2 direction in FIG. 3) toward the distal end side. Thus, when a proximal end of the outer multi-stripe hollow body 5 is rotated in a clockwise direction (C1 direction in FIG. 3), an outer diameter of the outer multi-stripe hollow body 5 becomes small.

By contrast, in the single-stripe hollow body 7, the metal element wire is wound in a clockwise direction (B1 direction in FIG. 2) toward the distal end side. Thus, when a proximal end of the single-stripe hollow body 7 is rotated in a clockwise direction (B1 direction in FIG. 2), an outer diameter of the single-stripe hollow body 7 becomes large.

However, the single-stripe hollow body 7 is of a single-stripe with a large twisting angle, and thus an amount by which the outer diameter increases when one end is rotated is small.

Here, the multilayer hollow body 1 illustrated in FIG. 4 will be referred to.

In FIG. 4, when the proximal end of the multilayer hollow body 1 including the inner multi-stripe hollow body 3, the single-stripe hollow body 7, and the outer multi-stripe hollow body 5 is rotated in a clockwise direction (D1 direction in FIG. 4), the outer diameter of the inner multi-stripe hollow body 3 becomes small, the outer diameter of the single-stripe hollow body 7 becomes large, and the outer diameter of the outer multi-stripe hollow body 5 becomes small. Consequently, the adhesion between the single-stripe hollow body 7 and the outer multi-stripe hollow body 5 is increased.

Meanwhile, when the proximal end of the multilayer hollow body 1 is rotated in a clockwise direction (D1 direction in FIG. 4), the outer diameter of the inner multi-stripe hollow body 3 becomes small, and the outer diameter of the single-stripe hollow body 7 becomes large, so that the space between the inner multi-stripe hollow body 3 and the single-stripe hollow body 7 tends to increase. However, the single-stripe hollow body 7 is of a single-stripe with a large twisting angle, and thus an amount by which the space increases is small.

Therefore, when the proximal end of the multilayer hollow body 1 is rotated in a clockwise direction (D1 direction in FIG. 4), it is possible to improve rotational torque transmissivity to the distal end of the multilayer hollow body 1.

### 2. Case of rotating a proximal end of the hollow multilayer body 1 in a counterclockwise direction

In the inner multi-stripe hollow body 3, the metal element wires are wound in a counterclockwise direction (A2 direction in FIG. 1) toward the distal end side. Thus, when the proximal end of the inner multi-stripe hollow body 3 is rotated in a counterclockwise direction (A2 direction in FIG. 1), an outer diameter of the inner multi-stripe hollow body 3 becomes large.

Also in the outer multi-stripe hollow body 5, the metal element wires are wound in a counterclockwise direction (C2 direction in FIG. 3) toward the distal end side. Thus, when the proximal end of the outer multi-stripe hollow body 5 is rotated in a counterclockwise direction (C2 direction in FIG. 3), an outer diameter of the outer multi-stripe hollow body 5 becomes large.

Meanwhile, in the single-stripe hollow body 7, the metal element wire is wound in a clockwise direction (B1 direction in FIG. 2) toward the distal end side. Thus, when the proximal end of the single-stripe hollow body 7 is rotated in a counterclockwise direction (B2 direction in FIG. 2), an outer diameter of the single-stripe hollow body 7 becomes small.

However, the single-stripe hollow body 7 is of a single-stripe with a large twisting angle, and thus an amount by which the outer diameter reduces when one end is rotated is small.

Here, the multilayer hollow body 1 illustrated in FIG. 4 will be referred to.

In FIG. 4, when the proximal end of the multilayer hollow body 1 including the inner multi-stripe hollow body 3, the single-stripe hollow body 7, and the outer multi-stripe hollow body 5 is rotated in a counterclockwise direction (D2 direction in FIG. 4), the outer diameter of the inner multi-stripe hollow body 3 becomes large, the outer diameter of the single-stripe hollow body 7 becomes small, and the outer diameter of the outer multi-stripe hollow body 5 becomes large. Consequently, the adhesion between the single-stripe hollow body 7 and the inner multi-stripe hollow body 3 is increased.

Meanwhile, when the proximal end of the multilayer hollow body 1 is rotated in a counterclockwise direction (D2 direction in FIG. 4), the outer diameter of the outer multi-stripe hollow body 5 becomes large, and the outer diameter of the single-stripe hollow body 7 becomes small, so that the space between the outer multi-stripe hollow body 5 and the single-stripe hollow body 7 tends to increase. However, the single-stripe hollow body 7 is of a single-stripe with a large twisting angle, and thus an amount by which the space increases is small.

Therefore, when the proximal end of the multilayer hollow body 1 is rotated in a counterclockwise direction (D2 direction in FIG. 4), it is possible to improve rotational torque transmissivity to the distal end of the multilayer hollow body 1.

Note that a reason why the rotational torque transmissivity of the multi-stripe body 4 is improved is the same as the above-described reason as to why the rotational torque transmissivity of the multilayer hollow body 1 is improved, and thus the explanation thereof is omitted.

With the multilayer hollow body 1 and the multi-stripe body 4 of the embodiment, the adhesion between the single-stripe hollow body 7 and the inner multi-stripe hollow body 3 is increased, and the space between the single-stripe hollow body 7 and the outer multi-stripe hollow body 5 is reduced as much as possible, or the adhesion between the single-stripe hollow body 7 and the outer multi-stripe hollow body 5 is increased, and the space between the single-stripe hollow body 7 and the inner multi-stripe hollow body 3 is reduced as much as possible, whereby when one end of the multilayer hollow body 1 and the multi-stripe body 4 is rotated in both a clockwise direction and a counterclockwise direction, it is possible to improve rotational torque transmissivity to the other end of the multilayer hollow body 1.

Note that the multilayer hollow body 1 is a three-stripe hollow body including the inner multi-stripe hollow body 3 and the outer multi-stripe hollow body 5 with winding in a same direction, and the single-stripe hollow body 7 arranged between the inner multi-stripe hollow body 3 and the outer multi-stripe hollow body 5 with winding in an opposite direction from the inner multi-stripe hollow body 3 and the outer multi-stripe hollow body 5. However, the multilayer hollow body 1 is not limited to the above described multilayer hollow body.

However, it is necessary to provide at least a one-stripe single-stripe hollow body, and two multi-stripe hollow bodies that are disposed adjacently to the inner side and the outer side of the single-stripe hollow body with winding in an opposite direction from the one-stripe single-stripe coil.

### (First embodiment)

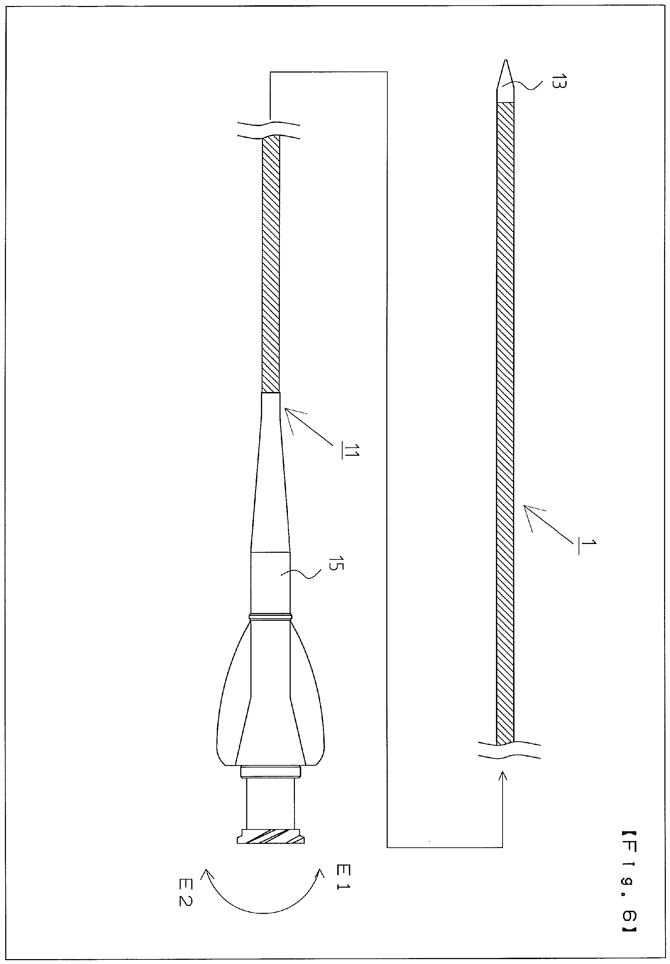

FIG. 6 is a schematic side view of a catheter according to the first embodiment of the present invention.

In FIG. 6, a catheter 11 includes the multilayer hollow body 1 described above, a distal end tip 13 connected to a distal end of the multilayer hollow body 1, and a connector 15 connected to a proximal end of the multilayer hollow body 1.

Note that regarding the multilayer hollow body 1 used in the embodiment, various multilayer hollow bodies can be applied, as described above in respect of the multilayer hollow body 1.

The distal end tip 13 includes a lumen (not illustrated) communicated to a lumen of the multilayer hollow body 1. The distal end of the distal end tip 13 has a tapered form thinner toward the distal end.

The material of the distal end tip 13 is metal, resin, or the like, and is not particularly limited. However, it is preferably metal when the material of the multilayer hollow body 1 is metal. With this, the distal end tip 13 and the multilayer hollow body 1 can be joined firmly by welding.

Moreover, the connector 15 also has a lumen communicated to the lumen of the multilayer hollow body 1. The material of the connector 15 is not particularly limited. However, it is normally formed of resin.

The catheter 11 of the embodiment includes the multilayer hollow body 1. Thus, when the connector 15 is rotated in a counterclockwise direction (E2 direction in FIG. 6), the adhesion between the single-stripe hollow body 7 and the inner multi-stripe hollow body 3 is increased, and the space between the single-stripe hollow body 7 and the outer multi-stripe hollow body 5 is reduced as much as possible, whereby it is possible to improve torque transmissivity to the distal end tip 13 of the catheter 11.

Moreover, when the connector 15 is rotated in a clockwise direction (E1 direction in FIG. 6), the adhesion between the single-stripe hollow body 7 and the outer multi-stripe hollow body 5 is increased, and the space between the single-stripe hollow body 7 and the inner multi-stripe hollow body 3 is reduced as much as possible, whereby it is possible to improve torque transmissivity to the distal end tip 13 of the catheter 11.

### (Second embodiment)

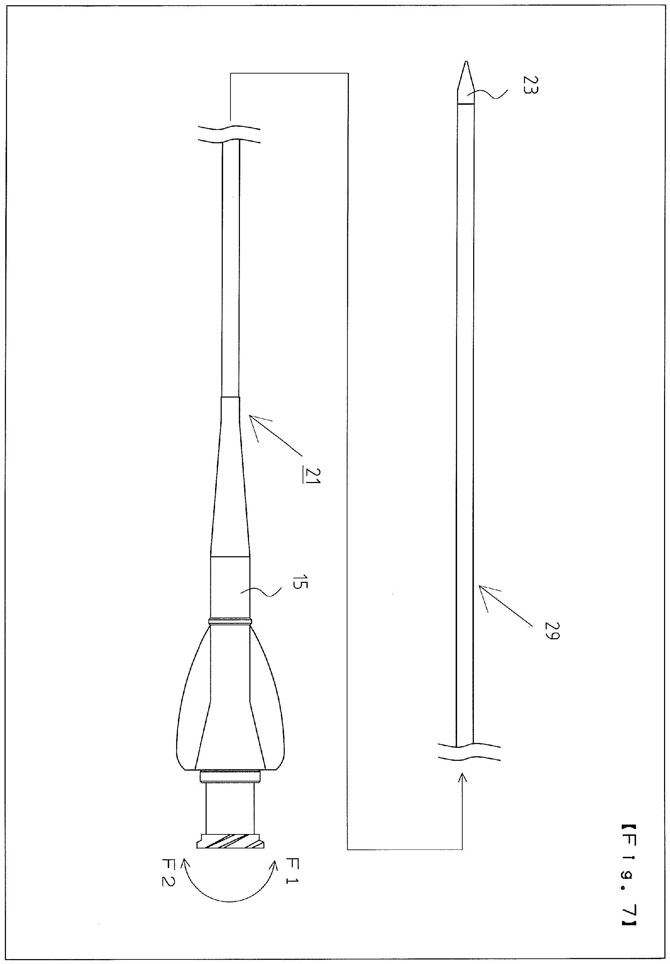

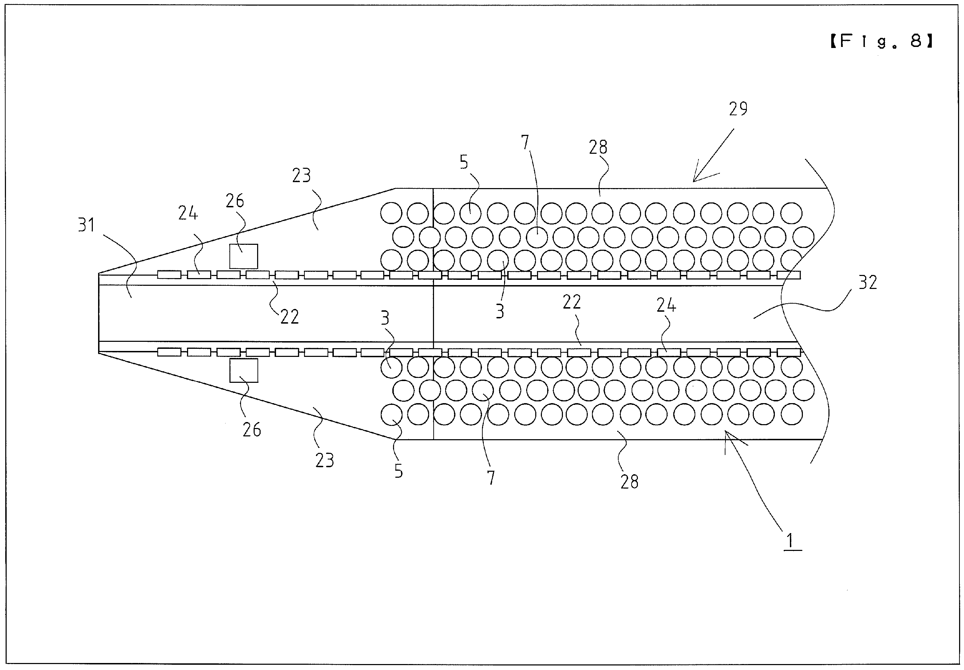

FIG. 7 is a schematic side view of a catheter according to a second embodiment of the present invention. FIG. 8 is a section view of a distal end of the catheter according to the second embodiment.

In FIG. 7, a catheter 21 includes a catheter main body 29, a distal end tip 23 connected to a distal end of the catheter main body 29, and the connector 15 connected to a proximal end of the catheter main body 29.

The catheter main body 29 includes, as illustrated in FIG. 8, an inner layer 22 that is a hollow cylindrical body having a lumen 32, a braid 24 covering an outer periphery of the inner layer 22, the multilayer hollow body 1 described above covering the braid 24, and an outer layer 28 covering the multilayer hollow body 1.

Note that regarding the multilayer hollow body 1 used in the embodiment, various multilayer hollow bodies can be applied, as described above in respect of the multilayer hollow body.

The distal end tip 23 includes a lumen 31 communicated to the lumen 32 of the multilayer hollow body 1. The distal end of the distal end tip 23 has a tapered form thinner toward the distal end.

The material of the distal end tip 23 is not particularly limited. However, in the embodiment, a material formed of resin softer than the catheter main body 29 is used.

Note that the inner layer 22 and the braid 24 extend to the distal end of the distal end tip 23 from the catheter main body 29. Moreover, the multilayer hollow body 1 also extends into the distal end tip 23 from the catheter main body 29.

Moreover, in the distal end tip 23, a marker 26 formed of a radiopaque material is disposed.

Furthermore, the connector 15 also has a lumen communicated to the lumen 32 of the multilayer hollow body 1. The material of the connector 15 is not particularly limited. However, it is normally formed of resin.

The catheter 21 of the embodiment includes the multilayer hollow body 1, and the multilayer hollow body 1 enters the inside of the distal end tip 23. Thus, when the connector 15 is rotated in a counterclockwise direction (F2 direction in FIG. 7), the adhesion between the single-stripe hollow body 7 and the inner multi-stripe hollow body 3 is increased, and the space between the single-stripe hollow body 7 and the outer multi-stripe hollow body 5 is reduced as much as possible, whereby it is possible to improve torque transmissivity to the distal end tip 23 of the catheter 21.

Moreover, when the connector 15 is rotated in a clockwise direction (F1 direction in FIG. 7), the adhesion between the single-stripe hollow body 7 and the outer multi-stripe hollow body 5 is increased, and the space between the single-stripe hollow body 7 and the inner multi-stripe hollow body 3 is reduced as much as possible, whereby it is possible to improve torque transmissivity to the distal end tip 23 of the catheter 21.

In the above-described embodiments, the element wire forming the multilayer hollow body is an element wire with a round section. However, it may be an element wire with a substantially rectangular section. However, when the multilayer hollow body itself appears on an outer surface, the element wire with a round section is preferable from the viewpoint of preventing a catch in the outside.

- 1: multilayer hollow body

- 2: element wire
- 3: inner multi-stripe hollow body
- 5: outer multi-stripe hollow body
- 7: single-stripe hollow body
- 11, 21: catheter
- 13, 23: distal end tip
- 15: connector
- 22: inner layer
- 24: braid
- 26: marker
- 28: outer layer
- 29: catheter main body
- 31, 32: lumen

## Claims

1. A catheter (11, 21), comprising:
a multilayer hollow body (1), comprising,
a single-stripe hollow body (7) that is wound by one element wire,
an inner hollow body (3) that is disposed adjacently to an inner periphery of the single-stripe hollow body (7), and
an outer hollow body (5) that is disposed adjacently to an outer periphery of the single-stripe hollow body (7); and
a distal end tip (13, 23) that is connected to a distal end of the multilayer hollow body (1); and
a connector (15) that is connected to a proximal end of the multilayer hollow body (1), **characterised in that**
the inner hollow body (3) is a multi-stripe hollow body that is wound by a plurality of element wires in an opposite direction from the single-stripe hollow body (7), and
the outer hollow body (5) is a multi-stripe hollow body that is wound by a plurality of element wires in an opposite direction from the single-stripe hollow body (7).

2. The catheter (21) according to claim 2, wherein the multilayer hollow body (1) enters an inside of the distal end tip (23).

3. The catheter (21) according to claim 1 or 2, further comprising:
an inner layer (22), wherein the multilayer hollow body (1) is disposed on an outer periphery of the inner layer (22); and
an outer layer (28) that covers the inner layer (22) and the multilayer hollow body (1), wherein:
the distal end tip (23) includes the distal end of the multilayer hollow body (1) inside the distal end tip (23) and is connected to a distal end of the inner layer (22) and a distal end of the outer layer (28); and
the connector (15) is connected to a proximal end of the inner layer (22), and a proximal end of the outer layer (28) .

## Patentansprüche

1. Katheter (11, 21) mit:
einem mehrschichtigem Hohlkörper (1), der aufweist:
einen aus einem Elementdraht gewickelten einstreifigen Hohlkörper (7),
einen am Innenumfang des einstreifigen Hohlkörpers (7) angeordneten inneren Hohlkörper (3) und
einen am Außenumfang des einstreifigen Hohlkörpers (7) angeordneten äußeren Hohlkörper (5);
einer distalen Spitze (13, 23), die mit einem distalen Ende des mehrschichtigen Hohlkörpers (1) verbunden ist; und
einem Verbinder (15), der mit einem proximalen Ende des mehrschichtigen Hohlkörpers (1) verbunden ist, **dadurch gekennzeichnet, dass**
der innere Hohlkörper (3) ein aus einer Vielzahl von Elementdrähten gegensinnig zum einstreifigen Hohlkörper (7) gewickelter mehrstreifiger Hohlkörper ist, und
der äußere Hohlkörper (5) ein aus einer Vielzahl von Elementdrähten gegensinnig zum einstreifigen Hohlkörper (7) gewickelter mehrstreifiger Hohlkörper ist.

2. Katheter (21) nach Anspruch 2, wobei der mehrschichtige Hohlkörper (1) in die distale Spitze (23) eindringt.

3. Katheter (21) nach Anspruch 1 oder 2, des Weiteren mit:
einer Innenschicht (22), wobei der mehrschichtige Hohlkörper (1) am Außenumfang der Innenschicht (22) angeordnet ist; and
einer Außenschicht (28), die die Innenschicht (22) und den mehrschichtigen Hohlkörper (1) ummantelt, wobei:
die distale Spitze (23) das distale Ende des mehrschichtigen Hohlkörpers (1) innerhalb der distalen Spitze (23) enthält und mit einem distalen Ende der Innenschicht (22) und einem distalen Ende der Außenschicht (28) verbunden ist; und
der Verbinder (15) mit einem proximalen Ende der Innenschicht (22) und einem proximalen Ende der Außenschicht (28) verbunden ist.

## Revendications

1. Cathéter (11, 21), comprenant :
un corps creux à plusieurs couches (1), comprenant,
un corps creux à une seule bande (7) autour duquel est enroulé un fil élémentaire,
un corps creux intérieur (3) qui est disposé de manière adjacente à une périphérie intérieure du corps creux à une seule bande (7), et
un corps creux extérieur (5) qui est disposé de manière adjacente à une périphérie extérieure du corps creux à une seule bande (7) ; et
une pointe d'extrémité distale (13, 23) qui est raccordée à une extrémité distale du corps creux à plusieurs couches (1) ; et
un raccord (15) qui est raccordé à une extrémité proximale du corps creux à plusieurs couches (1), **caractérisé en ce que**
le corps creux intérieur (3) est un corps creux à plusieurs bandes autour duquel sont enroulés une pluralité de fils élémentaires dans une direction opposée au corps creux à une seule bande (7), et
le corps creux extérieur (5) est un corps creux à plusieurs bandes autour duquel sont enroulés une pluralité de fils élémentaires dans une direction opposée au corps creux à une seule bande (7).

2. Cathéter (21) selon la revendication 2, dans lequel le corps creux à plusieurs couches (1) entre à l'intérieur de la pointe d'extrémité distale (23).

3. Cathéter (21) selon la revendication 1 ou 2, comprenant en outre :
une couche intérieure (22), dans lequel le corps creux à plusieurs couches (1) est disposé sur une périphérie extérieure de la couche intérieure (22) ; et
une couche extérieure (28) qui couvre la couche intérieure (22) et le corps creux à plusieurs couches (1), dans lequel :
la pointe d'extrémité distale (23) inclut l'extrémité distale du corps creux à plusieurs couches (1) dans la pointe d'extrémité distale (23) et est raccordée à une extrémité distale de la couche intérieure (22) et à une extrémité distale de la couche extérieure (28) ; et
le raccord (15) est raccordé à une extrémité proximale de la couche intérieure (22), et à une extrémité proximale de la couche extérieure (28).
